(19) Europäisches Patentamt
European Patent Office
Office européen des brevets

(11) Veröffentlichungsnummer: **0 189 071**
**A2**

(12) # EUROPÄISCHE PATENTANMELDUNG

(21) Anmeldenummer: **86100291.3**

(22) Anmeldetag: **11.01.86**

(51) Int. Cl.⁴: **C 07 D 513/04**, C 07 D 487/04, C 07 D 498/04
// (C07D513/04, 285:00, 235:00)

(30) Priorität: **24.01.85 DE 3502264**

(43) Veröffentlichungstag der Anmeldung: **30.07.86**
**Patentblatt 86/31**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Stubbe, Mathias, Dr., Zur Waldesruh 100, D-5600 Wuppertal 11 (DE)**
Erfinder: **Samaan, Samir, Dr., Claudiusweg 9, D-5600 Wuppertal 1 (DE)**

(54) **Imidazoalkensäurederivate, ein Verfahren zu ihrer Herstellung und ihre Verwendung für die Herstellung pharmazeutischer Wirkstoffe.**

(57) Imidazoalkensäurederivate der allgemeinen Formel I

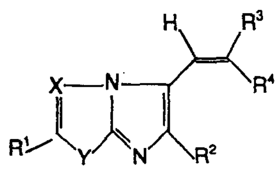

(I)

werden hergestellt, indem man Aldehyde der allgemeinen Formel II

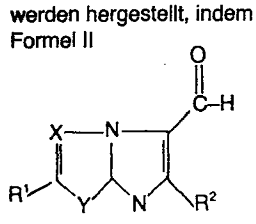

(II)

mit Malonsäurederivaten der allgemeinen Formel III

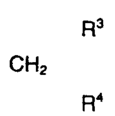

umsetzt. In den Formeln haben X, Y, $R^1$, $R^2$, $R^3$ und $R^4$ die in der Beschreibung angegebene Bedeutung. Die Verbindungen der Formel I sind Zwischenprodukte für die Herstellung vonn diuretischen Wirkstoffen.

EP 0 189 071 A2

ACTORUM AG

0189071

BAYER AKTIENGESELLSCHAFT
Konzernverwaltung RP
Patentabteilung
5090 Leverkusen, Bayerwerk
Si-klu/c

# Imidazoalkensäurederivate, ein Verfahren zu ihrer Herstellung und ihre Verwendung für die Herstellung pharmazeutischer Wirkstoffe

Die vorliegende Erfindung betrifft neue Imidazoalkensäurederivate, ein Verfahren zur ihrer Herstellung und ihre Verwendung als Zwischenprodukte für die Herstellung von Imidazoalkensäureamiden.

Imidazoalkensäureamide, Verfahren zu ihrer Herstellung und ihre Verwendung als Wirkstoffe von Arzneimitteln sind bereits bekannt geworden /DE-OS 3020421; DE-OS 3043158/.

Die erfindungsgemäßen Verbindungen sind Imidazoalkensäurederivate der allgemeinen Formel I

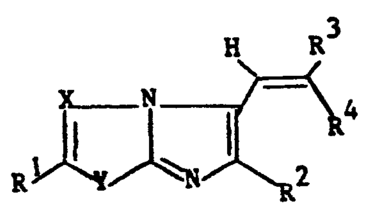

(I)

in welcher

X für N oder CH steht,

Y für S, O, NH oder N-$C_1$-$C_4$-Alkyl steht,

$R^1$ und $R^2$ gleich oder verschieden sein können und jeweils für Wasserstoff, geradkettiges, verzweigtes oder cyclisches, gegebenenfalls durch Phenyl, Cyano, Hydroxy oder Halogen substituiertes Alkyl, Alkenyl oder Alkinyl mit jeweils bis zu 6 C-Atomen, oder für einen Phenyl-, Naphthyl-, Furyl-, Thienyl-, Pyrimidyl-, Pyrazinyl-, Chinolyl-, Isochinolyl- oder Pyridylrest stehen, die jeweils substituiert sein können durch $C_1$-$C_4$-Alkyl, $C_1$-$C_4$-Alkoxy, Halogen, Nitro, Trifluormethyl oder $SO_n$-Alkyl (n = 0, 1 oder 2, Alkyl mit 1-4 C-Atomen),

$R^3$ und $R^4$ gleich oder verschieden sein können,

$R^3$ für CN oder COOR' steht, wobei R' Wasserstoff oder geradkettiges, verzweigtes oder cyclisches Alkyl mit 1-6 C-Atomen, Phenyl, Benzyl oder Naphthyl bedeutet,

$R^4$ für CN oder COOR" steht, wobei R" geradkettiges, verzweigtes oder cyclisches Alkyl mit 1-6 C-Atomen, Phenyl, Benzyl oder Naphthyl bedeutet,

oder $R^3$ und $R^4$ zusammen die Gruppierung

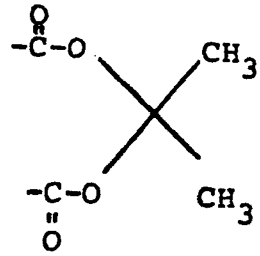

bedeuten.

Bevorzugt bedeuten $R^1$ und $R^2$ Alkylreste mit 1-4 C-Atomen, Phenyl oder Naphthyl; besonders bevorzugt bedeuten sie Methyl oder Phenyl.

Bevorzugt bedeutet $R^3$ CN oder COOR', wobei R' für Wasserstoff, oder geradkettiges oder verzweigtes Alkyl mit 1-4 C-Atomen steht; besonders bevorzugt steht $R^3$ für CN, COOH oder $COOC_2H_5$.

Bevorzugt steht $R^4$ für CN oder COOR", wobei R" für geradkettiges oder verzweigtes Alkyl mit 1-4 C-Atomen steht; besonders bevorzugt steht $R^4$ für CN oder $COOC_2H_5$.

Sowohl die Antipoden als auch die Racemformen als auch die Diasteromerengemische gehören zur vorliegenden Erfindung, ebenso die an der Doppelbindung der Seitenketten möglichen E/Z-Isomeren.

Die erfindungsgemäße Herstellung der erfindungsgemäßen Imidazoalkensäurederivate der allgemeinen

Formel I erfolgt durch Kondensation der aus DE-OS 3 043 158 bekannten Aldehyde der allgemeinen Formel II

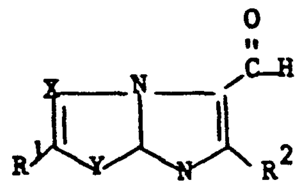

(II)

in der

$R^1$, $R^2$, X und Y die oben angegebene Bedeutung haben, mit Malonsäurederivaten der allgemeinen Formel III

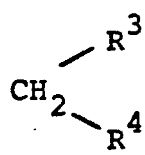

(III)

in der

$R^3$ und $R^4$ die oben angegebene Bedeutung haben.

Je nach Wahl der Ausgangsverbindungen können bei dem erfindungsgemäßen Verfahren die Verbindungen der allgemeinen Formel I in stereoisomeren Formen auftreten.

Die erfindungsgemäßen Umsetzungen können in Gegenwart von sauren und basischen Katalysatoren, wie sekundäre Amine und deren Salze, Mineralsäure und basische Alkali- und Erdalkalisalze, und gegebenenfalls in

Gegenwart inerter organischer Lösungsmittel bei Temperaturen von 20-200°C durchgeführt werden.

Bevorzugte Katalysatoren sind sekundäre und tertiäre Amine und deren Salze; besonders bevorzugt ist Piperidiniumacetat.

Als Lösungsmittel kommen alle protischen und aprotischen Lösungsmittel in Frage. Hierzu gehören halogenierte Kohlenwasserstoffe wie Dichlor-, Trichlormethan, Trichlorethan, Ether wie Diethylether, Dioxan, Tetrahydrofuran oder Glykolmono-, Glykoldimethylether, Kohlenwasserstoffe wie Toluol oder Xylol, Alkohole wie Ethanol, Isopropanol, tert.-Butanol, Dimethylformamid, Methylisobutylketon oder Acetonitril. Bevorzugtes Lösungsmittel ist Toluol.

Verwendet man als Ausgangsstoffe 5-Formyl-2-methyl-6-phenyl-imidazo -/2,1-b/-1,3,4-thiadiazol und Malonsäurediethylester, kann die Reaktion durch folgendes Formelschema beschrieben werden:

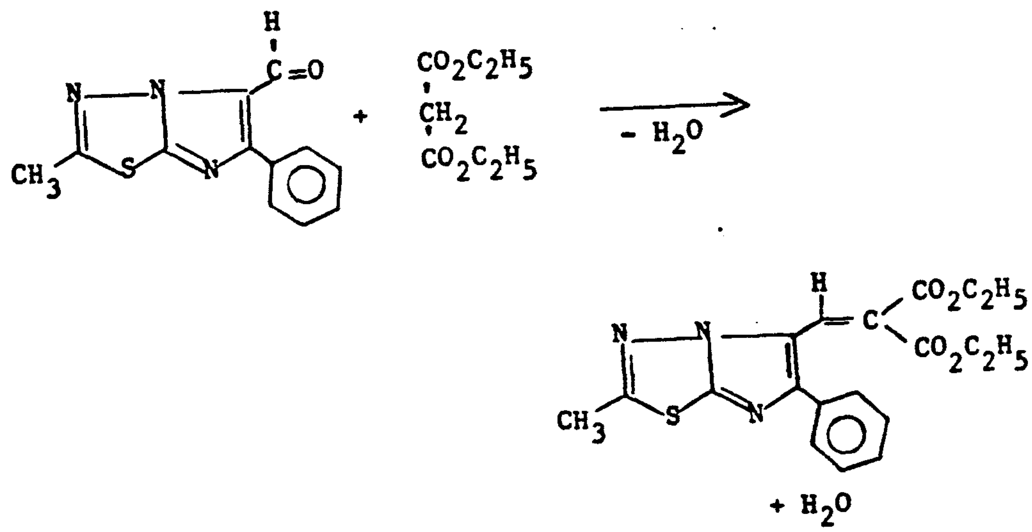

Bevorzugt arbeitet man mit Toluol in der Siedehitze. Dabei ist es vorteilhaft, das azeotrop abdestillierte Reaktionswasser von der Reaktionsmischung abzutrennen.

Die erfindungsgemäßen Verbindungen der allgemeinen Formel I können durch saure oder basische Hydrolyse in die entsprechenden Alkylidenmalonsäuren überführt werden, die spontan zu den entsprechenden aus DE-OS 3 043 158 bekannten Alkensäuren der allgemeinen Formel IV decarboxylieren,

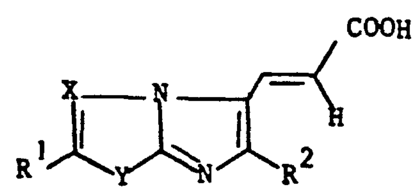

(IV)

wobei $R^1$, $R^2$, X und Y die oben angegebene Bedeuutng haben.

Für die Herstellung der Alkensäuren der allgemeinen Formel IV ist eine Isolierung und Reinigung der erfindungsgemäßen Verbindungen der allgemeinen Formel I oder der entsprechenden Malonsäuren nicht notwendig; es können vielmehr auch die Rohprodukte zur Hydrolyse und Decarboxylierung eingesetzt werden und die Isolierung und Reinigung auf der Stufe der Alkensäuren erfolgen.

Alkensäuren der allgemeinen Formel IV sind Ausgangsverbindungen für die Herstellung der entsprechenden

Imidazolalkensäureamide, die in DE-OS 3 020 421 und DE-OS 3 043 158 beschrieben sind. Diese sind hochwirksame diuretische Wirkstoffe.

Im Vergleich zu dem in DE-OS 3 043 158 und DE-OS 3 020 421 beschriebenen Herstellverfahren der Säuren der Formel IV aus dem entsprechenden Aldehyd der Formel II und Malonsäure in Pyridin bietet die erfindungsgemäße Verfahrensweise erhebliche Vorteile, da ein viel geringerer molarer Überschuß an Reagenz verwendet werden kann ( 11 % statt 57 % Überschuß).

Die zersetzliche und damit gefährliche Malonsäure wird durch stabilere Malonsäurederivate ersetzt, das giftige und geruchsbelästigende Pyridin wird durch das unbedenkliche Toluol ersetzt.

Beispiel 1

2-Methyl-6-phenyl-imidazo-/2,1-b/-1,3,4-thiadiazolyl-5-methylidenmalonsäure-diethylester

121,5 g 5-Formyl-2-methyl-6-phenyl-imidazo-/2,1-b/-1,3,4-thiadiazol, 80,0 g Malonsäurediethylester und 7,3 g Piperidiniumacetat in 150 ml Toluol werden 4 Stunden am Wasserabscheider gekocht (ca. 100°C). Man kühlt auf 5°C ab, rührt 1 Stunde nach, saugt ab, verrührt den Filterrückstand mit 200 ml Wasser, saugt ab und kristallisiert das Rohprodukt aus 200 ml Toluol um.
Ausbeute: 176,7 g ≙ 92 % der Theorie; Schmelzpunkt: 133-134°C.

Beispiel 2

ß-/2-Methyl-6-phenyl-imidazo-/2,1-b/-1,3,4-thiadiazolyl-5-/-acrylsäure

In 400 ml Toluol werden 300 g 5-Formyl-2-methyl-6-phenyl-imidazo-/2,1-b/-1,3,4-thiadiazol und 220 g Malonsäurediethylester mit 39 g Piperidiniumacetat 3 Stunden unter Rückfluß mit Wasserabscheidung gekocht. Das Lösungsmittel wird vollständig abdestilliert.

Der verbeibende Rückstand wird in 1,5 kg 50 proz. Schwefelsäure suspendiert und anschließend 8 Stunden bei 90-95°C gerührt. Es werden 750 ml Wasser zugegeben und nach dem Abkühlen auf 15-20°C abfiltriert.

Das Rohprodukt wird in 25 ltr. Wasser suspendiert und mit Natronlauge ein pH-Wert von 3,0 eingestellt. Es wird auf 90°C erhitzt, 30 Minuten bei dieser Temperatur nachgerührt und heiß filtriert. Das Produkt wird gut mit Wasser gewaschen und anschießend im Vakuum bei erhöhter Temperatur getrocknet.

Ausbeute: 299 g (85 %)
Fp.: 280-283°C (Zers.)

Beispiel 3

2-Methyl-6-phenyl-imidazo-[2,1-b]-1,3,4-thiadiazolyl-5-methylidenmalonsäuredinitril

Analog Beispiel 1 werden 50 g 5-Formyl-2-methyl-6-phenyl-imidazo-[2,1-b]-1,3,4-thiadiazol in 413,5 g Malonsäuredinitril umgesetzt.

Ausbeute: 55,6 g
Fp.: 250-252°C

Beispiel 4

α-Cyan-ß/2-Methyl-6-phenyl-imidazo -/2,1-b/-1,3,4-thiadiazolyl-5/-acrylsäureethylester

Analog Beispiel 1 werden 12,2 g 5-Formyl-2-methyl-6-phenyl-imidazo-/2,1-b/-1,3,4-thiadiazol mit 5,7 g Cyanessigsäureethylester umgesetzt.

Ausbeute: 15,0 g (89 % der Theorie)
Fp.: 167-168°C

Beispiel 5

ß-/2-Methyl-6-phenyl-imidazo -/2,1-b/-1,3,4-thiadiazolyl-5/-acrylsäureethylester

Analog Beispiel 1 werden 12,2 g 5-Formyl-2-methyl-6-phenyl-imidazo-/2,1-b/-1,3,4-thiadiazol mit 6,6 g Malonsäuremonoethylester umgesetzt. Unter diesen Bedingungen decarboxyliert der intermediär gebildete Alkylidenmalonester zum Acrylsäureester.

Ausbeute: 14,9 g (95 % der Theorie).

Beispiel 6

ß-2-Methyl-6-phenyl-imidazo-[2,1-b]-thiadiazolyl-5-acrylsäure

Analog Beispiel 2 werden 20 g 5-Formyl-2-methyl-6-phenyl-imidazo-[2,1-b]-1,3,4-thiadiazol zunächst mit 12 g Meldrumsäure (2,2-Dimethyl-1,3-dioxan-4,6-dion) in Toluol umgesetzt und anschließend in 50 proz. Schwefelsäure hydrolysiert.

Ausbeute: 19,9 g (85 % der Theorie)
Fp.: 279-282°C (Zers.).

Patentansprüche

1. Imidazoalkensäurederivate der allgemeinen Formel I

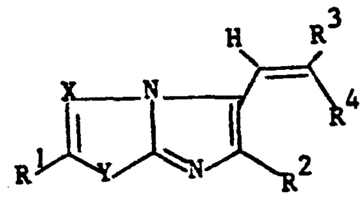

(I)

in welcher

X für N oder CH steht,

Y für S, O, NH oder N-$C_1$-$C_4$-Alkyl steht,

$R^1$ und $R^2$ gleich oder verschieden sein können und jeweils für Wasserstoff, geradkettiges, verzweigtes oder cyclisches, gegebenenfalls durch Phenyl, Cyano, Hydroxy oder Halogen substituiertes Alkyl, Alkenyl oder Alkinyl mit jeweils bis zu 6 C-Atomen, oder für einen Phenyl-, Naphthyl-, Furyl-, Thienyl-, Pyrimidyl-, Pyrazinyl-, Chinolyl-, Isochinolyl- oder Pyridylrest stehen, die jeweils substituiert sein können durch $C_1$-$C_4$-Alkyl, $C_1$-$C_4$-Alkoxy, Halogen, Nitro, Trifluormethyl oder $SO_n$-Alkyl (n = 0, 1 oder 2, Alkyl mit 1-4 C-Atomen),

$R^3$ und $R^4$ gleich oder verschieden sein können,

$R^3$ für CN oder COOR' steht, wobei R' Wasserstoff oder geradkettiges, verzweigtes oder cyclisches Alkyl mit 1-6 C-Atomen, Phenyl, Benzyl oder Naphthyl bedeutet,

$R^4$ für CN oder COOR" steht, wobei R" geradkettiges, verzweigtes oder cyclisches Alkyl mit 1-6 C-Atomen, Phenyl, Benzyl oder Naphthyl bedeutet,

oder $R^3$ und $R^4$ zusammen die Gruppierung

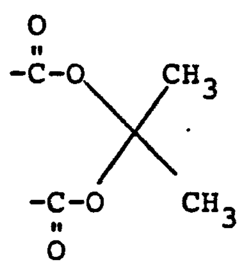

bedeuten.

2. Imidazolalkensäurederivate der allgemeinen Formel I in Anspruch 1,

in welcher

X und Y die oben angegebene Bedeutung haben,

$R^1$ und $R^2$ Alkylreste mit 1-4 C-Atomen, Phenyl oder Naphthyl bedeuten,

$R^3$ CN oder COOR' bedeutet, wobei R' für Wasserstoff oder geradkettiges oder verzweigtes Alkyl mit 1-4 C-Atomen steht, und

$R^4$ CN oder COOR" bedeutet, wobei R" für geradkettiges oder verzweigtes Alkyl mit 1-4 C-Atomen steht.

3. Imidazoalkensäurederivate der allgemeinen Formel I in Anspruch 1,

in welcher

X, Y, $R^1$ und $R^2$ die oben angegebene Bedeutung haben,

$R^3$ CN, COOH oder $COOC_2H_5$ und

$R^4$ CN oder $COOC_2H_5$

bedeutet.

4. 2-Methyl-6-phenyl-imidazo-[2,1-b]-1,3,4-thiadiazolyl-5-methyliden-malonsäure-diethylester.

5. Verfahren zur Herstellung von Imidazoalkensäurederivaten der allgemeinen Formel I

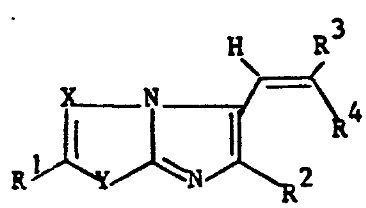

(I)

in welcher

X für N oder CH steht,

Y für S, O, NH oder N-$C_1$-$C_4$-Alkyl steht,

$R^1$ und $R^2$ gleich oder verschieden sein können und jeweils für Wasserstoff, geradkettiges, verzweigtes oder cyclisches, gegebenenfalls durch Phenyl, Cyano, Hydroxy oder Halogen substituiertes Alkyl, Alkenyl oder Alkinyl mit jeweils bis zu 6 C-Atomen, oder für einen Phenyl-, Naphthyl-, Furyl-, Thienyl-, Pyrimidyl-, Pyrazinyl-, Chinolyl-, Isochinolyl- oder Pyridylrest stehen, die jeweils substituiert sein können durch $C_1$-$C_4$-Alkyl, $C_1$-$C_4$-Alkoxy, Halogen, Nitro, Trifluormethyl oder $SO_n$-Alkyl (n = 0, 1 oder 2, Alkyl mit 1-4 C-Atomen),

$R^3$ und $R^4$ gleich oder verschieden sein können,

$R^3$ für CN oder COOR' steht, wobei R' Wasserstoff oder geradkettiges, verzweigtes oder cyclisches Alkyl mit 1-6 C-Atomen, Phenyl, Benzyl oder Naphthyl bedeutet,

$R^4$ für CN oder COOR" steht, wobei R" geradkettiges, verzweigtes oder cyclisches Alkyl

mit 1-6 C-Atomen, Phenyl, Benzyl oder Naphthyl bedeutet,

oder $R^3$ und $R^4$ zusammen die Gruppierung

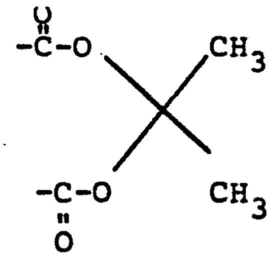

bedeuten,

dadurch gekennzeichnet, daß man Aldehyde der allgemeinen Formel II

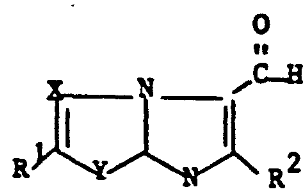

(II)

in der

$R^1$, $R^2$, X und Y die oben angegebene Bedeutung haben, mit Malonsäurederivaten der allgemeinen Formel III

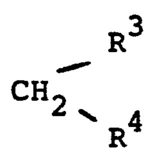

(III)

in der

$R^3$ und $R^4$ die oben angegebene Bedeutung haben,

kondensiert.

6. Verfahren zur Herstellung von Imidazoalkensäurederivaten nach Anspruch 5, dadurch gekennzeichnet, daß man die Kondensation in Gegenwart saurer oder basischer Katalysatoren durchführt.

7. Verfahren zur Herstellung von Imidazoalkensäurederivaten nach Anpruch 5, dadurch gekennzeichnet, daß man die Kondensation von in Gegenwart von Piperidiniumacetat durchführt.

8. Verfahren zur Herstellung von Imidazoalkensäurederivaten nach Anspruch 5, dadurch gekennzeichnet, daß man die Kondensation bei Temperaturen von 20-200°C durchführt.

9. Verfahren zur Herstellung von Imidazoalkensäurederivaten nach Anspruch 5, dadurch gekennzeichnet, daß man die Kondensation in einem protischen oder aprotischen Lösungsmittel durchführt.

10. Verwendung von Imidazoalkensäurederivaten der allgemeinen Formel I in Anspruch 1 als Zwischenprodukte für die Herstellung pharmazeutischer Wirkstoffe.